Europäisches Patentamt
European Patent Office
Office européen des brevets

(19)

(11) Numéro de publication: **0 057 116**
**A2**

(12)

# DEMANDE DE BREVET EUROPEEN

(21) Numéro de dépôt: 82400028.5

(22) Date de dépôt: 08.01.82

(51) Int. Cl.³: C 08 G 79/04
C 08 G 63/68, C 08 G 63/76
A 61 L 17/00, A 61 L 15/00

(30) Priorité: 26.01.81 FR 8101400

(43) Date de publication de la demande:
04.08.82 Bulletin 82/31

(84) Etats contractants désignés:
AT BE CH DE IT LI NL

(71) Demandeur: LIGATURES PETERS
11 bis, rue Chapon
F-93302 Aubervilliers Cedex(FR)

(71) Demandeur: LORCA MARIN S.A.
7 Avenue Alfonso Xsabie
Murcie(ES)

(71) Demandeur: SERAG-WIESSNER CATGUTFABRIEKEN G.m.b.H.
D-8674 Naila / Bayern(DE)

(72) Inventeur: Fauvarque, Jean-François
6, rue Joseph Bara
F-75006 Paris(FR)

(72) Inventeur: Dubuisson, Dominique née Juanes
170, Avenue St-Maurice
F-34250 Palavas(FR)

(74) Mandataire: Phélip, Bruno et al,
c/o Cabinet Harlé & Phélip 21, rue de La Rochefoucauld
F-75009 Paris(FR)

(54) **Polymères insolubles à biodégradabilité réglable à volonté, leurs procédés de préparation et biomatériaux les comprenant.**

(57) Polymères à biodégradabilité réglable à volonté

Ils sont constitués d'oligomères bifonctionnels réunis par pontage du type phosphate, de formule générale I

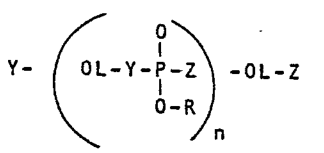

(1)

où : n ⩾ 2

OL représente un oligomère approprié

Y et Z représentent les deux fonctions de l'oligomère, lesquelles peuvent être identiques ou différentes, et qui peuvent être les groupes OH, $NH_2$, $NR_1H$, $R_1$ étant un groupe alkyle substitué ou non, et

R représente un cation, un atome d'hydrogène ou un groupe alkyle, aryle, aralkyle, substitués ou non.

Application à la réalisation de biomatériaux tels que fils, prothèses et pansements.

EP 0 057 116 A2

Polymères insolubles à biodégradabilité réglable à volonté,leurs procédés de préparation et biomatériaux les comprenant.

La présente invention est relative à de nouveaux polymères à biodégradabilité réglable à volonté,à leurs procédés de préparation et à leur application en tant que biomatériaux.

Le terme "biodégradabilité" employé dans la présente invention,est utilisé dans le sens le plus large, à savoir la faculté pour un polymère de pouvoir être hydrolysé et résorbé dans un milieu biologique,et notamment au sein d'un organisme vivant.

La qualité des biomatériaux utilisés dans le domaine médico-chirurgical (tels que fils pour sutures par exemple),a nettement progressé ces dernières années. Pendant très longtemps,ces fils ont été préparés à partir de catgut,lin,soie,argent,etc... Ces différents matériaux présentaient des inconvénients plus ou moins graves: s'ils étaient résorbables (comme par exemple le catgut),ils provoquaient des réactions défavorables dans l'organisme vivant,parfois même des complications graves consécutives à leurs caractéristiques antigéniques;d'autres matériaux, tels que les fils de lin,de soie ou d'argent,étaient peu ou pas du tout résorbables. Aussi, de très nombreux produits de remplacement à base de polymères synthétiques ont été proposés. Ainsi,ont été préconisés par exemple:

-des polymères et copolymères d'acide lactique [brevets français 1.478.694 et 1.478 695(1966)],

-des polyesters de β-hydroxyacides [brevet américain 3.225.766 (1962 )],

-des esters polyhydroxyacétiques [brevet français 1.412.957(1966)]

-des amino-triazoles polymères /¯brevet américain 3.809.683 (1973)_7
-des copolyoxalates /¯brevet américain 4.141 087 (1979)_7 et d'autres matériaux encore.

Ces différents biomatériaux présentent des propriétés uniformes,peuvent être stérilisés,sont absorbés par les tissus vivants,sont facilement manipulables. Leurs caractéristiques mécaniques sont toutefois variables;plus ou moins bonne solidité au nouage,plus ou moins bonne résistance à la traction. Certains des biomatériaux proposés sont difficilement filables,aucun des biomatériaux de l'art antérieur ne présente une biodégradabilité variable et réglable à volonté.

La présente invention s'est par conséquent donné pour but de pourvoir à un procédé et à un biomatériau qui répondent mieux aux nécessités de la pratique que les procédés et les biomatériaux de l'art antérieur,notamment en ce qu'ils réunissent à la fois:
-une très bonne faculté de filage et de façonnage,
-de très bonnes caractéristiques de manipulation,
-une très bonne solidité initiale,et la faculté de former des noeuds chirurgicaux tenant parfaitement
-et une biocompatibilité totale,ainsi qu'une biodégradabilité variable et réglable à volonté.

La présente invention a pour objet des polymères insolubles à biodégradabilité réglable à volonté, caractérisés en ce qu'ils sont constitués d'oligomères bifonctionneles réunis par pontage du type phosphate de formule générale I ci-après:

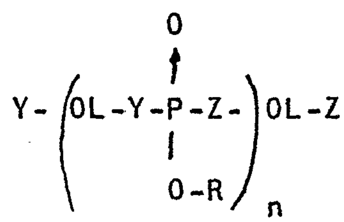

(I)

où : $n \geqslant 2$

OL représente un oligomère approprié

Y et Z , représentent les deux fonctions de l'oligomère,lesquelles peuvent être identiques ou différentes, et qui peuvent être les groupes OH, $NH_2$, $NR_1H$, $R_1$ étant un groupe alkyle substitué ou non, et

R représente un cation,un atome d'hydrogène ou un groupe alkyle, aryle ou aralkyle, substitués ou non.

Suivant un mode de réalisation avantageux de l'objet de l'invention,les oligomères OL formant le polymère sont identiques.

Suivant un autre mode de réalisation avantageux de l'objet de l'invention,les oligomères formant le polymère sont de nature différente.

En alliant les ponts phosphate avec des oligomères choisis pour leurs qualités mécaniques,la demanderesse a pu réaliser des polymères biodégradables de qualité exceptionnelle,satisfaisant à tous les critères exigibles des polymères utilisés dans un domaine aussi délicat qu'est le domaine médico- chirurgical.

Conformément à l'invention,les oligomères sont choisis de préférence dans le groupe qui comprend le polyéthylène-téréphtalate (PET),et le polybutylènetéréphtalate (PBT).

La présente invention a également pour objet un procédé de préparation des polymères à biodégradabilité réglable conformes à l'invention,caractérisé en ce

qu'on fait réagir sur un oligomère bifonctionnel,un agent de phosphorylation,et en ce qu'on procède à la polycondensation et à l'élimination de l'excès de l'agent de phosphorylation.

Conformément à l'invention,on fait par exemple réagir sur l'oligomère bifonctionnel en quantités au moins stoechiométriques,le phosphodichlorure

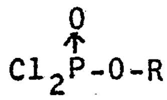

pour obtenir le produit de formule II ci-après:

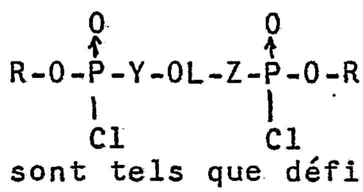
(II)

où:

Y, Z et R sont tels que définis précédemment.

Suivant un mode de réalisation avantageux de l'objet de l'invention,l'agent de phosphorylation formant le pont phosphate est constitué par le composé de formule III ci-après:

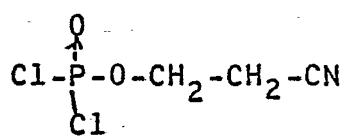
(III)

obtenu en traitant le cyanoéthanol par un oxytrihalogénure de phosphore,suivant la réaction

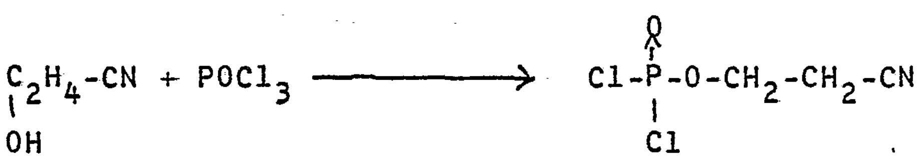
(IV)

Il suffit de remplacer ensuite le groupe $-CH_2-CH_2-CN$ par un groupe R convenablement choisi(le choix de R dépendant du degré et de la rapidité d'hydrolyse et de résorption désirés.

Suivant un mode de réalisation avantageux de procédé objet de la présente invention,la polycondensation est effectuée dans un solvant à haut point

d'ébullition,la température,la pression et la durée de la réaction étant fonction du nombre de ponts susceptibles d'être hydrolysés que l'on désire obtenir.

Suivant une modalité particulière de ce mode de réalisation,la polycondensation est effectuée en présence d'un catalyseur pris dans le groupe qui comprend le tétrabutylate de titane et l'oxyde d'antimoine.

La présente invention a également pour objet,les produits insolubles utilisables en tant que biomatériaux, tels que fils, prothèses,pansements,obtenus et façonnés à partir de polymères conformes à l'invention.

Outre les dispositions qui précèdent,l'invention comprend encore d'autres dispositions,qui ressortiront de la description qui va suivre.

La présente invention vise particulièrement les procédés de préparation de polymères à biodégradabilité variable et réglable à volonté, les polymères et les biomatériaux obtenus,ainsi que les moyens propres à la mise en oeuvre de ces procédés,les procédés d'ensemble et les chaînes de fabrication dans lesquels sont inclus les procédés conformes à la présente invention,de même que les objets constitués par, ou contenant,les polymères conformes à la présente invention.

L'invention pourra être mieux comprises à l'aide du complément de description qui va suivre,qui se réfère à des exemples de préparation et à des caractéristiques de polymères conformes à la présente invention.

Il doit être bien entendu, toutefois, que les différents exemples et caractéristiques sont donnés uniquement à titre d'illustration de l'objet de l'invention,mais n'en constituent en aucune manière une limitation.

EXEMPLE 1- SYNTHESE DES OLIGOMERES(PET)

Synthèse du monomère: dans un ballon tricol de 500 $cm^3$,on mélange 105g de diméthyltéréphtalate recristallisé dans le méthanol,150g d'éthylèneglycol distillé sur sodium,155 mg d'acétate de calcium et 20 $cm^3$ de méthanol. On monte la température à 140-150°C sous azote en 30 minutes et on maintient à cette température pendant 30 minutes. On chauffe ensuite le mélange à 180°C pendant 4 heures. On élimine l'excès d'éthylèneglycol par distillation. On laisse refroidir,on ajoute un catalyseur et on chauffe sous agitation pour homogénéiser.

Synthèse de l'oligomère: on coule le mélange précédent à l'état fondu dans le réacteur de polycondensation. On maintient le mélange à 285°C,sous vide poussé,pendant un temps variable, pour avoir l'oligomère de masse voulue.

Le tableau I ci-dessous donne le rapport de la masse moléculaire $Mn^{*}$ obtenue en fonction des conditions opératoires (les oligomères proviennent du même stock de monomères).

TABLEAU I

| Conditions | $Mn^{*}$ |
|---|---|
| 2 h sous 1 mm à 285°C | 3500 |
| 2 h sous 0,1 mm à 285°C | 4800 |
| 3 h 30 sous 0,1 mm à 285°C | 5600 |
| $\frac{1}{2}$ h sous 20 mm à 285°C | 1700 |
| $\frac{1}{2}$ h sous 10 mm et 1 h sous 0,5 mm à 285°C | 3800 |

$Mn^{*}$ est mesuré par viscosimétrie dans l'o-chlorophénol à 25°C $Mn^{*} = 38000\,[\eta]^{1,3}$.

EXEMPLE 2-SYNTHESE DU CYANOETHYLPHOSPHODICHLORURE

On ajoute le 3-hydroxypropionitrile à un très large excès d'oxychlorure de phosphore à 0°C. On maintient deux heures à 0°C,puis on élimine l'excès de $POCl_3$ et on distille le réactif à 30°C sous 0,1 mm. Rendement : 40%

RMN 2 triplets 2,8 ppm et 3,8 ppm.

IR Bande CN 2250 $cm^{-1}$.

EXEMPLE 3-SYNTHESE DE PET RELIES PAR DES PONTS CYANOETHYLPHOSPHATE

60g d'oligomère polyéthylènetéréphtalate (Mn= 2300)sont traités par 11,5 g de cyanoéthylphosphodichlorure une nuit à 80°C, puis 7 heures sous 30 mm de Hg à 90°C. On filtre le solvant et on sèche le TEP diphosphoryle. On ajoute un solvant lourd tel que le bromonaphtalène et on procède à la polycondensation dans un tube à 270°C pendant une heure sous vide progressif jusqu'à 20 mm de Hg, puis pendant 3 h 30 sous 0,1 mm de Hg. On obtient un polymère de Mn = 22000.

Spectre RMN $^{31}P$ 1 pic à 1,65 ppm.

Un polymère de ce type a été filé. Pour Mn=13000, la résistance à la traction est supérieure à 10g pour un diamètre de 80 µ.

EXEMPLE 4-SYNTHESE DE PET RELIES PAR DES PONTS PHOSPHATE SEL DE SODIUM

Le produit obtenu dans l'exemple précédent, est traité par une base dans un solvant polaire,tel que le tertioamylate de sodium dans l'HMPT en quantité stoechiométrique,pendant une heure à 80°C. Le produit final est précipité par addition d'heptane. On vérifie que dans cette étape,la masse moléculaire moyenne ne varie pas sensiblement.

TABLEAU II

HYDROLYSE DU POLYMERE SOUS FORME D'UNE POUDRE ET D'UN FIL IN VITRO

L'hydrolyse a été conduite à 80°C dans le tampon de Michaelis, pH= 7,3.Elle a été contrôlée par la mesure de masse

| Objet | Formule | Masse moléculaire | Masse moléculaire après |
|---|---|---|---|
| Poudre | 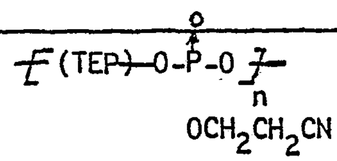 | Mn = 8300 | 10 jours Mn = 5000 |
| | 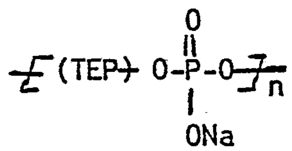 | Mn = 8300 | 3 jours Mn = 5000 |
| Fil(diamètre: 80 μ | 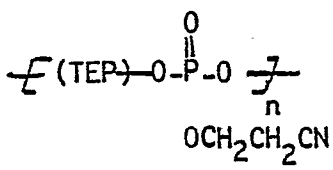 | Mn = 13000 | 4 jours Mn = 9300 |

Ainsi que cela ressort de ce qui précède,l'invention ne se limite nullement à ceux de ses modes de mise en oeuvre,de réalisation et d'application qui viennent d'être décrits de façon plus explicite;elle en embrasse au contraire toutes les variantes qui peuvent venir à l'esprit du technicien en la matière, sans s'écarter du cadre,ni de la portée de la présente invention.

## REVENDICATIONS

1.Polymères à biodégradabilité réglable à volonté,caractérisés en ce qu'ils sont constitués d'oligomères bifonctionnels réunis par pontage du type phosphate,de formule générale I

$$Y-\left( OL-Y-\underset{\mid \atop O-R}{\overset{O \atop \uparrow}{P}}-Z \right)_n - OL-Z \qquad (I)$$

où= $n \geqslant 2$

OL représente un oligomère approprié

Y et Z représentent les deux fonctions de l'oligomère,lesquelles peuvent être identiques ou différentes,et qui peuvent être les groupes OH,$NH_2$, $NR_1H$, $R_1$ étant un groupe alkyle substitué ou non, et

R représente un cation,un atome d'hydrogène ou un groupe alkyle,aryle ou aralkyle,substitués ou non.

2.Polymère selon la revendication 1,caractérisé en ce que les oligomères OL formant le polymère,sont identiques.

3.Polymère selon la revendication 1,caractérisé en ce que les oligomères formant le polymère,sont de nature différente.

4. Polymère selon l'une quelconque des revendications 1 à 3,caractérisé en ce que les oligomères sont choisis dans le groupe qui comprend le polyéthylènetéréphtalate (PET) et le polybutylènetéréphtalate (PBT).

5. Procédé de préparation des polymères selon l'une quelconque des revendications 1 à 4,caractérisé en ce qu'on fait réagir sur un oligomère bifonctionnel,un agent de phosphorylation et en ce qu'on procède à la polycondensation et à l'élimination de l'excès de l'agent de phosphorylation.

6.Procédé selon la revendication 5,caractérisé en ce qu'on fait réagir sur l'oligomère bifonctionnel,en des quantités au moins stoechiométriques, le phosphodichlorure

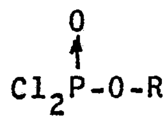

pour obtenir le produit de formule II ci-après:

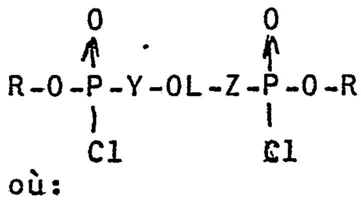 (II)

où:

Y, Z et R sont tels que définis précédemment.

7.Procédé selon la revendication 5,caractérisé en ce que l'agent de phosphorylation formant le pont phosphate est constitué par le composé de formule III ci-après:

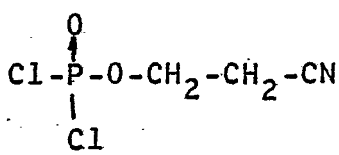 (III)

obtenu en traitant le cyanoéthanol par un oxytrihalogénure de phosphate,suivant la réaction:

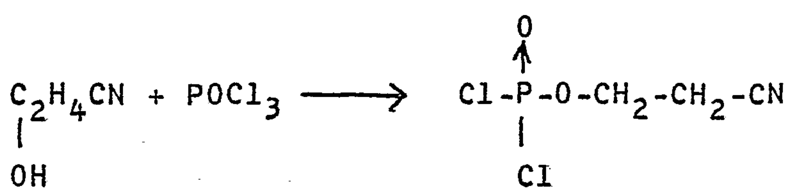 (IV)

8.Procédé selon l'une quelconque des revendications 5 à 7,caractérisé en ce que la polycondensation est effectuée en présence d'un catalyseur pris dans le groupe qui comprend le tétrabutylate de titane et l'oxyde d'antimoine.

9.Biomatériaux,tels que fils,prothèses et pansements,caractérisés en ce qu'ils comprennent des polymères selon l'une quelconque des revendications 1 à 4.